# EUROPÄISCHE PATENTSCHRIFT

(11) **EP 0 897 977 B1**
(45) Veröffentlichungstag und Bekanntmachung des Hinweises auf die Patenterteilung: **03.03.2004**
(21) Anmeldenummer: 98115157.4
(22) Anmeldetag: 12.08.1998
(51) Int. Cl.: C12N 9/24, C12N 1/14

(54) **Enzymkomplex**
Enzyme complex
Complexe enzymatique

(30) Priorität: 21.08.1997 EP 97114431
(43) Veröffentlichungstag der Anmeldung: 24.02.1999
(73) Patentinhaber: F. HOFFMANN-LA ROCHE AG, 4070 Basel (CH)
(72) Erfinder: Ringpfeil, Manfred, 12621 Berlin (DE)
(74) Vertreter: Keller, Günter, Dr.

(56) Entgegenhaltungen:
- EP-A- 0 455 928
- DD-A- 291 673
- DE-A- 3 404 470
- ZEILINGER SUSANNE ET AL: "Different inducibility of expression of the two xylanase genes xyn1 and xyn2 in Trichoderma reesei." JOURNAL OF BIOLOGICAL CHEMISTRY, Bd. 271, Nr. 41, 1996, Seiten 25624-25629, XP002196924 ISSN: 0021-9258

## Beschreibung

Die vorliegende Erfindung betrifft ein fermentatives Verfahren zur Herstellung Xylanase-reicher Enzymkomplexe unter Verwendung vorbehandelter Kornbrennereischlempe als Induktor der Xylanasebildung.

Bekanntlich ist Xylanase die Bezeichnung für ein den Hemicellulasen zuzuordnendes Enzym, das Xylane (oder "Holzgummi") zu Xylose und anderen Zuckern hydrolysiert. Das Enzym ist auch als Endo-1,4-β-D-Xylanase oder 1,4-β-D-Xylan-Xylanohydrolase bekannt und gehört der Enzymklasse EC 3.2.1.8 an. Xylane selber, die Polysaccharide aus 1,4-β-glykosid-verknüpften D-Xylopyranosen mit kurzen, unterschiedlich zusammengesetzten Seitenketten sind und im Molekül auch Arabinose, Glucose, Galactose und/oder Glucuronsäure sowie Acetyl- und Methylgruppen enthalten, sind Bestandteile vieler Laub- und Nadelhölzer sowie von Getreide, Kleie, Pektin, Tragant, Pflanzengummi usw. Aufgrund ihres Vorkommens in Holz gehören Xylane zu den weitverbreitesten Naturstoffen. Angesichts ihrer Strukturvielfalt erfordert der vollständige Abbau verzweigter, teilweise acetylierter Xylane die Wirkung verschiedener Xylanasen, durch welche die Xylane zu Xylose und anderen Zuckern hydrolysiert werden. Diese Einwirkung von Xylanasen ist komplex und wird immer zusammen mit (z.T. synergistisch wirkenden) anderen Enzymen realisiert.

Xylanasen werden von Pilzen, z.B. *Trichoderma*, *Penicillium*, *Aspergillus*, *Talaromyces* und *Sporotrichum*, und Bakterien, z.B. *Clostridium*, *Cellulomonas, Bacillus, Thermononspora* und *Ruminococcus*, gebildet. Sie werden in der Zellstoffindustrie vor allem als Bleich- und Veredelungsmittel und neuerdings bei der Tierfutterherstellung verwendet. In bezug auf das letztere Anwendungsgebiet weisen Untersuchungen über den Einsatz exogener Enzyme, z.B. von Xylanase, in Roggen, Gerste oder Triticale enthaltenden Futtermitteln auf einen günstigen Einfluss der diesbezüglichen Präparate auf die Reduktion der antinutritiven Wirkung der Nichtstärke-Polysaccharide und auf eine verbesserte Verdaulichkeit und Absorption der Nährstoffe im Tierdarm hin. Die wichtigste Gruppe von Enzymen, die heutzutage insbesondere in der Broilerzucht Verwendung finden, besteht gerade aus denjenigen Enzymen, welche die in Getreidesorten, wie beispielsweise Gerste, Weizen und Roggen, vorhandenen Nichtstärke-Polysaccharide hydrolysieren können. Es befinden sich bereits mehrere solche Enzyme enthaltende Präparate auf dem Markt, wie beispielsweise Roxazyme® G (Roche), das Cellulase, β-Glucanase und Xylanase als die Hauptenzyme enthält. Solche Präparate werden dem Tierfutter wegen der oben erwähnten Vorteile bei der Tierfütterung beigemischt; die erfindungsgemäss hergestellten Xylanase-reichen Enzymkomplexe dienen als sehr nützliche Materialien zur Zubereitung dieser Präparate. Der Einsatz von Xylanase als Futterzusatz in der Geflügelernährung stellt einen wichtigen Anwendungsbereich dar, wobei beispielsweise ein wesentlich erhöhter Nährwert von Broilerfutter erbracht wird gegenüber demjenigen, der mit energiearmen Getreidearten, wie Gerste, Hafer, Roggen und Triticale, ohne einen solchen Futterzusatz erzielt wird [siehe Mh. Vet.-Med. 48, 213-217 (1993) und die darin zitierten Referenzen].

Wie oben erwähnt, betrifft die vorliegende Erfindung ein Verfahren zur Herstellung der besagten Enzymkomplexe, und zwar durch Kultivierung eines Xylanase-erzeugenden Mikroorganismus der Gattung *Trichoderma* in einem Nährmedium, das grundsätzlich Kohlenstoffquellen, Stickstoffquellen und bestimmte Salze enthält, und Isolierung des gebildeten Xylanase-reichen (und zugleich Cellulase- sowie β-Glucanase-verminderten) Enzymkomplexes vom Nährmedium. Insbesondere Mikroorganismen der Gattung *Trichoderma* gelten als Bildner polysaccharolytischer Aktivität bei eindeutiger Bevorzugung der cellulolytischen (d.h. die Aktivität richtet sich bevorzugt auf den Abbau der Cellulose). Es hat viele Versuche zur Veränderung dieses Aktivitätsspektrums gegeben, u.a. mit dem Ziel, die xylanolytische Aktivität selektiv zu erhöhen. Dazu hat man dem Nährmedium (Fermentationsmedium) xylanhaltige Stoffe zugesetzt, um eine Induktion der Xylanasebildung zu erreichen. Gereinigte Xylane, Weizenkleie, Gerstenspelzen, gemahlene Maiskolben (Maisspindelmehl), Stroh und Kornbrennereischlempe sind Beispiele dafür. Damit ist es gelungen, das Aktivitätsspektrum in Richtung einer höheren Xylanaseaktivität zu verschieben [siehe als technologischen Hintergrund die Deutschen Patentschriften DD 278 359 und DD 291 673; die Europäische Patentpublikation (EP) 0 455 928 A1; Appl. Microbiol. Biotechnol. 40, 224-229 (1993); sowie Enzyme & Microb. Technol. 18, 495-501 (1996)]. Besonders mit Kornbrennereischlempe lässt sich diese Induktorwirkung kostengünstig erzielen; Kornbrennereischlempe zeigt jedoch mit steigenden Konzentrationen eine inhibierende Wirkung auf das Wachstum und die Enzymbildung des jeweiligen Mikrooorganismus.

Ueberraschenderweise wurde nun gefunden, dass eine spezielle Vorbehandlung der als Induktor verwendeten Kornbrennereischlempe durch Verdampfung von Wasser und anderen flüchtigen Bestandteilen die oben erwähnte nachteilige inhibierende Wirkung der nativen Kornbrennereischlempe aufhob und die Induktorwirkung dabei steigerte, so dass die so behandelte konzentrierte Form der Kornbrennereischlempe vorteilhaft zur Anwendung gelangt. Die als Folge der Behandlung induzierte, während der Kultivierung des Mikroorganismus (im Fermentor) realisierte Enzymbildung bringt eine wesentliche Veränderung des Enzymspektrums hervor: die xylanolytische Aktivität übertrifft die Aktivität der anderen Enzyme, d.h. die cellulolytische und glucanolytische Aktivität, um mehrere Hundertprozente: eine überraschende Verschiebung des Verhältnisses Xylanase zu Cellulase zugunsten von Xylanase wird erzielt.

Der vorliegenden Erfindung liegt die Aufgabe zugrunde, einen Xylanase-reichen (und zugleich Cellulase- und β-Glucanase-verminderten) Enzymkomplex durch Kultivierung eines Xylanase-erzeugenden Mikroorganismus der Gattung *Trichoderma* in einem spezifischen Nährmedium herzustellen.

Diese Aufgabe wird erfindungsgemäss dadurch gelöst, dass als Mittel zur Induzierung der Xylanase-Bildung (nachfolgend als "Xylanase-Induktor" bezeichnet) und zugleich als Kohlenstoffquelle eine vorbehandelte Kornbrennereischlempe in der Kultivierung verwendet wird, wobei die Vorbehandlung in einer Abtrennung der Feststoffanteile der Kornbrennereischlempe, einer Konzentrierung der nichtflüchtigen Inhaltsstoffe durch Verdampfung von Wasser und anderen flüchtigen Substanzen sowie einer anschliessenden Autoklavierung des daraus resultierenden Kornbrennereischlempe-Konzentrates besteht.

Als Xylanase-erzeugende Mikroorganismen der Gattung *Trichoderma*, die im erfindungsgemässen Verfahren zur Herstellung eines Xylanase-reichen Enzymkomplexes verwendet werden, kommen insbesondere *Trichoderma* (T.) *reesei*-Mutanten in Frage, die sich aus dem Wildstamm QM6a ableiten, z.B. QM 9123, QM 9414, M 18.2, M 18.2-y, Rut M-7 und Rut NG-14 (siehe Bland S. Montenecourt, "Trichoderma reesei cellulases", Trends in Biotechnology, Vol. 1, No. 5, Seiten 156-160, 1983 und die darin zitierten Referenzen).

Einige der oben erwähnten Mikroorganismen-Stämme sind hinterlegt, und zwar

| | |
|---|---|
| QM6a | ATCC 13631, CCM F-560, CMI 45548, DSM 768; |
| QM 9123 | ATCC 24449; |
| QM 9414 | ATCC 26921, CCM F-522, DSM 769; |
| M 18.2 | DSM 10683; sowie |
| M18.2-y | DSM 7537. |

Die Mikroorganismen-Stämme *T*. *reesei* QM 6a, QM 9123 und QM 9414 sind in den Katalogen von internationalen Hinterlegungsstellen, z.B. der Deutschen Sammlung von Mikroorganismen und Zellkulturen GmbH (DSM) und/oder der American Type Culture Collection (ATCC), aufgelistet und demzufolge von diesen und weiteren Quellen gewerblich erhältlich. Der Mikroorganismen-Stamm M 18.2 wurde am 14. Mai 1996 von der Biopract GmbH, Rudower Chaussee 5, D-12489 Berlin bei der DSM zur Langzeiterhaltung hinterlegt und erhielt die Hinterlegungsnr. DSM 10683; am 12. Juni 1998 erhielt die DSM von der Biopract GmbH einen Antrag zur Umwandlung dieser Hinterlegung in eine Hinterlegung gemäss Budapester Vertrag. Schliesslich wurde der Mikroorganismen-Stamm M 18.2-y ursprünglich am 31. August 1989 gemäss Budapester Vertrag bei dem Zentralinstitut für mikrobielle und experimentelle Therapie (ZIMET) in Jena hinterlegt, und zwar unter der Hinterlegungsnr. IMET 43915. Am 4.März 1993 wurde dieser Stamm zur DSM in Braunschweig transferiert, wo er die neue Hinterlegungsnr. DSM 7537 erhielt. Infolge Eigentumsübertragungen erhielt schiesslich die F. Hoffmann-La Roche AG das Eigentumsrecht am Mikroorganismen-Stamm *T*. *reesei* M 18.2-y.

Die im erfindungsgemässen Verfahren als Xylanase-Induktor und zugleich als Kohlenstoffquelle verwendete vorbehandelte Kornbrennereischlempe stammt aus einer von Kornbrennereien erhältlichen Schlempe, die normalerweise als Abfallprodukt betrachtet wird. Die (unbehandelte) Kornbrennereischlempe entsteht bei der Branntweinproduktion aus Roggen auf folgende bekannte Weise: Auf der Grundlage von Roggen, Wasser und Enzymen (Exo- und Endoglucanasen) erfolgt eine Vergärung der Roggenstärke zu Alkohol mittels Hefen über drei Tage bei etwa 30°C. Nach Abdestillation des Alkohols verbleibt die weitgehend von Roggenstärke freie Mischung als Kornbrennereischlempe.

Die Inhaltsstoffe der Kornbrennereischlempe aus unterschiedlichen Ansätzen unterliegen den bei Naturprodukten üblichen Schwankungen und setzen sich überwiegend aus Kohlenhydraten (Cellulose, Hemicellulose, Pentosane), geringeren Anteilen an Eiweissen, Mineralstoffen, Fetten sowie Restmengen von Ethanol und anderen Produkten der Hefegärung zusammen.

Bei der Vorbehandlung der Kornbrennereischlempe handelt es sich im wesentlichen um die Abtrennung der Feststoffe, die Konzentrierung der nichtflüchtigen Inhaltsstoffe und die Autoklavierung des erhaltenen Konzentrates. Diese Vorbehandlungsstufen weisen folgende bevorzugte Merkmale auf:
- Abtrennung der Feststoffanteile der Kornbrennereischlempe durch Dekantieren und Separieren mittels eines Tellerseparators (z.B. des Typs SA 1, Westfalia Separator AG, Oelde, Deutschland). Das Volumen der zu verwerfenden feststoffhaltigen Fraktion beträgt etwa 30% bis etwa 50% des Volumens der Kornbrennereischlempe.
- Konzentrierung der nichtflüchtigen Inhaltsstoffe der feststofffreien Kornbrennereischlempe mittels eines Vakuum-Umlauf-Verdampfers (z.B. des Typs VUV 20-I, Schott & Gen, Jena, Deutschland) bei einer zwischen etwa 40°C und etwa 50°C liegenden Siedetemperatur und bei dem der Temperatur entsprechenden Dampfdruck der feststofffreien Kornbrennereischlempe. Unter diesen Bedingungen wird eine gewünschte Volumenverminderung der feststofffreien Kornbrennereischlempe um etwa 80% bis etwa 90% nach etwa einer Stunde erreicht. Die Konzentrierung der nichtflüchtigen Inhaltsstoffe beträgt dementsprechend etwa 1:5 bis etwa 1:10.
- Autoklavierung der konzentierten, feststofffreien Kornbrennereischlempe für mindestens etwa 30 Minuten bei etwa 121°C (zweckmässigerweise 121 ± 1°C).

Nicht nur die vorbehandelte Kornbrennereischlempe, sondern auch weitere Kohlenstoffquellen kommen für das Nährmedium im erfindungsgemässen Verfahren in Frage, und zwar u.a. Lactose, Cellulose, Xylane, Weizenkleie, Glucosesirup und sprühgetrocknetes Maisquellwasser.

Besonders bevorzugte weitere Kohlenstoffquellen sind Lactose, Cellulose und Haferspelzen-Xylan.

Als Stickstoffquellen für das Nährmedium kommen in Frage u.a. Ammoniumsulfat [(NH₄)₂SO₄], Ammoniak-Wasser [NH₃-H₂O] und entöltes Sojamehl (als organische Stickstoffquelle). Bezogen auf die Kohlenstoffquelle beträgt der Stickstoffanteil zweckmässigerweise mindestens 20%.

Als Phosphorquelle für das Nährmedium kommt in Frage z.B. Kaliumdihydrogenphosphat (KH₂PO₄). Bezogen auf die Kohlenstoffquelle beträgt der Phosphoranteil zweckmässigerweise mindestens 2,5%.

Das Nährmedium enthält zweckmässigerweise weitere Salze, und als solche kommen u.a. Magnesium-, Eisen-, Mangan- und Zinksulfat sowie Calcium- und Cobaltchlorid in Frage.

Als weiterer Bestandteil des Nährmediums kann beispielsweise ein Antischaummittel, z.B. M-30 (Serva Feinbiochemica, Heidelberg, Deutschland), Glanapon DG 102 (Bussetti, Wien, Oesterreich) oder MAZU 8005 (Quest International, Cork, Irland), verwendet werden.

Man verwendet im erfindungsgemässen Verfahren zweckmässigerweise etwa 35 bis etwa 45 g/l, vorzugsweise etwa 40 g/l, einer zehnfach konzentierten vorbehandelten Kornbrennereischlempe bezogen auf das Gesamtvolumen des Kulturmediums. Das Volumen des Inokulums für *Trichoderma reesei* beträgt zweckmässigerweise etwa 10% des Gesamtvolumens des Kulturmediums.

Der pH-Wert für die Kultivierung liegt zweckmässigerweise im Bereich von etwa 5,5 bis etwa 6,5, vorzugsweise bei etwa 6,0. Die Einstellung des pH-Wertes im Kulturmedium erfolgt zweckmässigerweise mit Ammoniak-Wasser in einer Konzentration von etwa 12,5% bis etwa 25% (bezogen auf NH₃).

Was die Kultivierungstemperatur betrifft, so beträgt diese zweckmässigerweise etwa 28°C bis etwa 34°C, vorzugsweise etwa 31°C.

Man kann als Fermentationsapparatur ein ganz übliches Kultivierungsgefäss (Fermentor) verwenden, bei dem für den Ausschluss von Fremdinfektionen vorgesorgt ist. Solche Kultivierungsgefässe weisen normalerweise u.a. eine Rührvorrichtung und eine Begasungsvorrichtung für die Luftzufuhr auf, da es sich bei der gemäss der Erfindung durchgeführten Kultivierung um eine aerobe Submerskultur des Mikroorganismus handelt. Die Rührerdrehzahl und Luftzufuhr sind zweckmässigerweise so zu regeln, dass der Sauerstoffgehalt im Kulturmedium nicht unter etwa 10% des Sauerstoffsättigungswertes abfällt.

Es eignet sich insbesondere die sogenannte "fed batch-Technik" zur Durchführung des erfindungsgemässen Verfahrens, wobei es sich im ersten Abschnitt (batch-Phase) um eine batch-Fermentation, gefolgt von einem Substratzuführungsabschnitt (fed batch-Phase) handelt.

Die batch-Phase dient der Anzucht von Mycel im Nährmedium, wobei das Animpfen (Inokulierung) des Fermentors zweckmässigerweise in drei Stufen (i)-(iii) erfolgt:
(i) Abspülen der Konidien einer Schrägagarkultur aus der Stammhaltung der Mikroorganismen mit sterilisiertem Leitungswasser. Das Wasser enthält zweckmässigerweise einen Emulgator, z.B. Tween® 80 (vorzugsweise in diesem Fall bei einer Konzentration von etwa 1 g/l). Die Konidiensuspension wird geeigneterweise auf mindestens 5 x 10⁸ Konidien/l, insbesondere 5 x 10⁸ - 1 x 10⁹ Konidien/l, eingestellt.
(ii) Mit der Konidiensuspension nach Stufe (i) werden Schüttelkolben, die zweckmässigerweise bekannte Nährmedien für Pilze der Gattung *Trichoderma reesei* auf der Grundlage von Glucose oder Cellulose als Kohlenstoffquelle, Phosphat, Nitrat, Harnstoff, Pepton und Spurenelementen enthalten, angeimpft [siehe beispielsweise R. Haapala, E. Parkkinen, P. Suominen und S. Linko, "Production of extracellular enzymes by immobilized *Trichoderma reesei* in shake flask cultures", Appl. Microbiol. Biotechnol. 43, 815-821 (1995)]. Das Verhältnis der Volumenteile Konidiensuspension zu Nährmedium in den Schüttelkolben beträgt zweckmässigerweise etwa 1:25 bis etwa 1:50. Die Konidien werden in diesen Schüttelkolben bei etwa 31°C ± 1°C (30-32°C), vorzugsweise bei etwa 31°C, und einer Schüttelfrequenz von etwa 200 bis etwa 300 UpM, vorzugsweise etwa 250 UpM, solange kultiviert, bis sich ein Kulturmedium mit gut verzweigtem Mycel ausgebildet hat. Es sollte noch keine Sporenbildung zu erkennen sein.
(iii) Mit dem Kulturmedium nach Stufe (ii) wird der Fermentor angeimpft, wobei das Verhältnis der Volumenanteile Kulturmedium zu Nährmedium im Fermentor zweckmässigerweise etwa 1:5 bis etwa 1:10, vorzugsweise etwa 1:5, beträgt.

In der anschliessenden fed batch-Phase erfolgt hauptsächlich die Enzymbildung. Die Zugabe der Substratlösung (Substratzuführung), vorzugsweise von vorbehandelter Kornbrennereischlempe als Induktor, von entöltem Sojamehl oder Sojamehlsud als organische Stickstoffquelle sowie von Lactose als Kohlenstoffquelle, wird geeigneterweise mittels einer Regeleinrichtung oder einer rechnergestützten Prozesssteuerung von der spezifischen Kohlendioxidentwicklung des Mycels angesteuert. Die Substratzuführung kann beispielsweise mittels der Kohlendioxidkonzentration des Fermentationsabgases angesteuert oder nach einem empirisch ermittelten Zeitregime vorgenommen werden.

Gemäss einem weiteren Aspekt der vorliegenden Erfindung wird entöltes Sojamehl oder Sojamehlsud als (zusätzliche) Stickstoffquelle verwendet. Es ist festgestellt worden, dass durch den Zusatz von entöltem Sojamehl oder von Sojamehlsud zur vorbehandelten Kornbrennereischlempe eine weitere Steigerung der Xylanaseproduktion erzielt wird. Demnach wird angenommen, dass auch das entölte Sojamehl oder der Sojamehlsud die Wirkung eines Xylanase-Induktors aufweist.

Bei dem entölten Sojamehl handelt es sich zweckmässigerweise um kommerzielle Produkte von Sojamühlen. Die Konzentrationen des entölten Sojamehls im Kulturmedium sind in der batch-Phase zweckmässigerweise etwa 20 bis 30 g/l Nährmedium und in der fed batch-Phase zweckmässigerweise etwa 15 bis 25 g/l Substratlösung, vorzugsweise etwa 25 g/l bzw. etwa 20 g/l.

Bei dem alternativ verwendeten Sojamehlsud handelt es sich um eine wässrige Lösung von Inhaltsstoffen des entölten Sojamehls. Sojamehlsud wird durch Suspendierung von entöltem Sojamehl in Wasser (etwa 35 g/l) gewonnen. Die Suspension wird etwa 10 Minuten gekocht, und nach Abkühlung auf Raumtemperatur werden die Feststoffanteile abfiltriert. Das Volumen des feststofffreien Filtrates (Sojamehlsud) beträgt etwa 80% des Volumens der Sojamehlsuspension vor der Filtration. Sojamehlsud wird alternativ zum entölten Sojamehl vorzugsweise in der fed batch-Phase verwendet, wobei das Volumen des Sojamehlsuds der Einwaage an entöltem Sojamehl entspricht.

Die Abtrennung des erfindungsgemäss gewonnenen Enzymkomplexes und dessen Reinigung und Konzentrierung können nach an sich bekannten Methoden vorgenommen werden. Grundsätzliche Anforderungen hierfür sind niedrige Medientemperaturen (etwa 5°C bis etwa 15°C) und niedrige pH-Werte (etwa 4 bis etwa 4,5) sowie aseptische Bedingungen. Die Prozedur beinhaltet zweckmässigerweise:
- Abtrennung der Biomasse aus dem Fermentationsmedium mittels Zentrifugation oder Filtration sowie Mikrofiltration;
- Konzentrierung des Enzymkomplexes mittels Ultrafiltration;
- Zur Herstellung eines Enzym-Feststoffpräparates kann der Konzentrierung des Enzymkomplexes eine Sprühtrocknung angeschlossen werden.

Hauptanwendungsgebiet des erfindungsgemäss gewonnenen Enzymkomplexes ist seine Verwertung als Futtermittelzusatz bei der Tierfutterherstellung. Der Enzymkomplex kann als nichtaufgearbeitetes Fermentationsmedium, als Kulturfiltrat, als Enzymkonzentrat oder als Feststoffpräparat eingesetzt werden.

Die vorliegende Erfindung wird durch das nachfolgende Beispiel veranschaulicht.

### Beispiel

Ein Xylanase-reicher Enzymkomplex wurde mit *Trichoderma reesei* M 18.2-y (DSM 7537) in einer fed batch-Fermentation hergestellt.

Zur Herstellung des Kornbrennereischlempe-Konzentrats wurde die feststofffreie Fraktion einer Kornbrennereischlempe unter Vakuum bei etwa 50°C um das 7,5-fache aufkonzentriert und etwa 30 Minuten bei 121°C autoklaviert.

Zur Herstellung des Nährmediums für die batch-Phase wurden 1800 ml Nährmedium bestehend aus 20,8 g/l Lactose, 8,3 g/l mikrokristalliner Cellulose, 25 g/l entöltem Sojamehl, 33,3 ml/l Kornbrennereischlempe-Konzentrat, 3,75 g/l KH₂PO₄, 6,0 g/l (NH₄)₂SO₄, 0,5 g/l MgSO₄•7H₂O, 0,5 g/l CaCl₂•2H₂O, 6,25 mg/l FeSO₄•7H₂O, 2,0 mg/l MnSO₄•H₂O, 1,75 mg/l ZnSO₄•7H₂O und 2,5 mg/l CoCl₂•6H₂O in einem 5 l Fermentor 20 Minuten bei 121°C autoklaviert und mit 12,5% Ammoniak-Wasser auf pH 6,0 eingestellt.

Zur Herstellung der Substratlösung für die fed batch-Phase wurden 1000 ml der Substrat- und Induktorlösung bestehend aus 300 g/l Lactose, 10 g/l Cellulose, 500 ml/l Sojamehlsud und 107 ml/l Kornbrennereischlempe-Konzentrat 20 Minuten bei 121°C autoklaviert. (Zur Herstellung der 500 ml Sojamehlsud wurden 20 g Sojamehl in 600 ml Wasser suspendiert; die Suspension wurde 10 Minuten gekocht und der Feststoffanteil abfiltriert.)

In der batch-Phase wurde der Fermentor mit 300 ml Schüttelkolben-Vorkultur inokuliert. Zur Herstellung des diesbezüglichen Inokulums wurden zunächst in zwei Schrägagarröhrchen aus der Stammhaltung jeweils etwa 3 ml sterilisiertes Leitungswasser mit 1 g/l Tween® 80 (Polyethoxysorbitanoleat) eingefüllt und die Konidien von beiden Agaroberflächen abgeschüttelt. Im folgenden Schritt wurden drei Schüttelkolben, die jeweils 100 ml Nährmedium enthielten, mit der Konidiensuspension zu gleichen Teilen angeimpft. Das Nährmedium setzte sich zusammen aus 20 g/l Glucose, 15,0 g/l KH₂PO₄, 4,8 g/l (NH₄)₂SO₄, 0,3 g/l CaCl₂•2 H₂O, 0,3 g/l MgSO₄•7 H₂O, 5,0 mg/l FeSO₄•7 H₂O, 1,6 mg/l MnSO₄•H₂O, 1,4 mg/l ZnSO₄•7 H₂O und 2,0 mg/l CoCl₂•6 H₂O. Anschliessend wurden die Schüttelkolbenvorkulturen bei 31°C und einer Schüttelfrequenz von etwa 250 UpM kultiviert. Der pH-Wert des Mediums wurde nicht eingestellt und nicht geregelt. Zu Beginn der Kultivierung betrug der pH-Wert etwa 5, am ende der Kultivierung etwa 3,5. Nach einer Kultivierungsdauer von 28 Stunden wurde der Fermentor mit diesen Schüttelkolben-Vorkulturen angeimpft. Danach wurde mit 12% Ammoniak-Wasser ein pH-Wert von 6,0 eingestellt. Der Sauerstoffgehalt wurde mittels Rührerkaskadenregelung auf etwa 10% des Sauerstoffsättigungswertes bei 31°C gehalten. Nach 21 Stunden Kultivierung wurde ein erstes CO₂-Maximum im Fermentor-Abgas überschritten, und damit das Ende der batch-Phase erreicht.

Im Rahmen der fed batch-Phase wurde nach Absinken des CO₂-Gehaltes im Abgas auf 80% des erstens Maximums mit der diskontinuierlichen Zugabe der Substratlösung begonnen. Jede Zugabe führte zu einem weiteren CO₂-Maximum. Die Zugaben erfolgten portionensweise automatisch nach Unterschreiten des CO₂-Gehaltes im Abgas auf 80% des vorhergehenden Maximums. Die zugegebenen Volumen der Substratlösung entsprachen einer Zugabe von 1,5 g Lactose per Liter Kulturmedium und von 2,0 g Lactose per Liter Kulturmedium ab 50 Stunden Fermentation.

Die Fermentation wurde nach 72 Stunden durch Abkühlen auf 10°C und Einstellen des pH-Wertes auf 4,5 beendet. Das Mycel wurde abfiltriert und die verbliebene Fermentationslösung der Analyse unterworfen. Die Enzymaktivitäten im Fermentationsmedium betrugen:

| | |
|---|---|
| Xylanase | 2625 U/ml |
| β-Glucanase | 555 U/ml |
| Carboxymethylcellulase (CMCase) | 330 U/ml |

Die Konzentration der gelösten Proteine im Fermentationsmedium betrug 17,0 g/l.

### Enzym- und Proteinbestimmungen

Die Aktivität der Xylanase (Endo-1,4-β-xylanase) wurde durch Inkubieren mit einer 0,5%igen Xylansuspension (Xylan aus Haferspelzen, Roth) in 40 mM Natriumacetat-Puffer (pH 6,0) 20 Minuten bei 50°C bestimmt. Eine Einheit (U) von Xylanase setzt 1 µmol Xylose pro Minute frei.

Die Aktivität der β-Glucanase (Endo-1,3-1,4-β-glucanase) wurde durch Inkubieren mit einer 0,5% Licheninsuspension (Lichenin, Carl Roth GmbH, Karlsruhe, Deutschland) in 40 mM Natriumacetat-Puffer (pH 6,0) 20 Minuten bei 50°C bestimmt.

Die Aktivität der CMCase (Endo-1,4-β-glucanase) wurde durch Inkubieren mit einer 2,0%igen Lösung von Carboxymethylcellulose-Natriumsalz (Carl Roth GmbH) in 40 mM Natriumacetat-Puffer (pH 6,0) 20 Minuten bei 50°C bestimmt. Eine Einheit (U) von β-Glucanase bzw. CMCase setzt 1 µmol Glucose pro Minute frei.

Die Proteinbestimmung wurde im Anschluss an eine Proteinfällung (Zugabe von Trichloressigsäure) mittels einer modifizierten Methode nach Lowry auf der Basis von Bicinchoninsäure durgeführt: Sigma Procedure No. TPRO-562 (Sigma Chemical Co., St. Louis, USA).

## Patentansprüche

1. Verfahren zur Herstellung eines Xylanase-reichen Enzymkomplexes durch Kultivierung eines Xylanase-erzeugenden Mikroorganismus der Gattung *Trichoderma* in einem Nährmedium, **dadurch gekennzeichnet, dass** als Xylanase-Induktor und zugleich als Kohlenstoffquelle eine vorbehandelte Kornbrennereischlempe in der Kultivierung verwendet wird, wobei die Vorbehandlung in einer Abtrennung der Feststoffanteile der Kornbrennereischlempe, einer Konzentrierung der nichtflüchtigen Inhaltsstoffe durch Verdampfung von Wasser und anderen flüchtigen Substanzen sowie einer anschliessenden Autoklavierung des daraus resultierenden Kornbrennereischlempe-Konzentrates besteht.

2. Verfahren nach Anspruch 1, **dadurch gekennzeichnet, dass** die Abtrennung der Feststoffanteile der Kornbrennereischlempe durch Dekantieren und Separieren mittels eines Tellerseparators erfolgt, wobei das Volumen der zu verwerfenden feststoffhaltigen Fraktion etwa 30% bis etwa 50% des Volumens der Kornbrennereischlempe beträgt.

3. Verfahren nach Anspruch 1 oder 2, **dadurch gekennzeichnet, dass** die Konzentrierung der nichtflüchtigen Inhaltsstoffe der feststofffreien Kornbrennereischlempe mittels eines Vakuum-Umlauf-Verdampfers bei einer zwischen etwa 40°C und etwa 50°C liegenden Siedetemperatur und bei dem der Temperatur entsprechenden Dampfdruck der feststofffreien Kornbrennereischlempe erfolgt, wobei eine gewünschte Volumen-veminderung der feststofffreien Kornbrennereischlempe um etwa 80% bis etwa 90% nach etwa einer Stunde erreicht wird.

4. Verfahren nach einem der Ansprüche 1 bis 3, **dadurch gekennzeichnet, dass** die Autoklavierung des Kornbrennereischlempe-Konzentrats für mindestens etwa 30 Minuten bei etwa 121°C durchgeführt wird.

5. Verfahren nach einem Ansprüche 1 bis 4, **dadurch gekennzeichnet, dass** als zu kultivierender Xylanase-erzeugender Mikroorganismus eine *Trichoderma reesei*-Mutante, die sich aus dem Wildstamm QM6a ableitet, insbesondere QM 9123, QM 9414, M 18.2, M 18.2-y, Rut M-7 oder Rut NG-14, verwendet wird.

6. Verfahren nach einem der Ansprüche 1 bis 5, **dadurch gekennzeichnet, dass** als weitere Kohlenstoffquellen für das Nährmedium Lactose, Cellulose, Xylane, Weizenkleie, Glucosesirup und sprühgetrocknetes Maisquellwasser, vorzugsweise Lactose, Cellulose und Haferspelzen-Xylan, verwendet werden.

7. Verfahren nach einem der Ansprüche 1 bis 6, **dadurch gekennzeichnet, dass** als Stickstoffquellen für das Nährmedium Ammoniumsulfat, Ammoniak-Wasser und entöltes Sojamehl verwendet werden.

8. Verfahren nach einem der Ansprüche 1 bis 7, **dadurch gekennzeichnet, dass** das Nährmedium auch eine Phosphorquelle, vorzugsweise Kaliumdihydrogenphosphat, und/oder Salze, vorzugsweise Magnesium-, Eisen-, Mangan- und Zinksulfat sowie Calcium- und Cobaltchlorid, aufweist.

9. Verfahren nach einem der Ansprüche 1 bis 8, **dadurch gekennzeichnet, dass** der pH-Wert und die Temperatur für die Kultivierung im Bereich von etwa 5,5 bis etwa 6,5 bzw. im Bereich von etwa 28°C bis etwa 34°C liegt.

10. Verfahren nach einem der Ansprüche 1 bis 9, **dadurch gekennzeichnet, dass** als zusätzlicher Xylanase-Induktor und als Stickstoffquelle entöltes Sojamehl oder Sojamehlsud verwendet wird.

11. Verfahren nach einem der Ansprüche 1 bis 10, **dadurch gekennzeichnet, dass** zur Durchführung des Verfahrens die fed batch-Technik angewendet wird.

## Claims

1. A process for the manufacture of a xylanase-rich enzyme complex by the cultivation of a xylanase-producing microorganism of the genus *Trichoderma* in a nutrient medium, which process comprises using in the cultivation a pre-treated thin stillage of rye as the xylanase inductor and simultaneously as the carbon source, with the pre-treatment comprising a removal of the solid constituents of the thin stillage of rye, a concentration of the non-volatile components by evaporation of water and other volatile substances as well as a subsequent autoclaving of the thin stillage of rye concentrate resulting therefrom.

2. A process according to claim 1, wherein the removal of the solid constituents of the thin stillage of rye is carried out by decantation and separation using a plate separator, with the volume of the solid-containing fraction to be discarded amounting to about 30% to about 50% of the volume of the thin stillage of rye.

3. A process according to claim 1 or 2, wherein the concentration of the non-volatile components of the solid-free thin stillage of rye is carried out using a rotary vacuum evaporator at a boiling temperature lying between about 40°C and about 50°C and at the vapour pressure of the solid-free thin stillage of rye corresponding to the temperature, with a desired volume reduction of the solid-free thin stillage of rye by about 80% to about 90% being achieved after about one hour.

4. A process according to any one of claims 1 to 3, wherein the autoclaving of the thin stillage of rye is carried out for at least about 30 minutes at about 121°C.

5. A process according to any one of claims 1 to 4, wherein a *Trichoderma reesei* mutant, which is derived from the wild strain QM6a, especially QM 9123, QM 9414, M 18.2, M 18.2-y, Rut M-7 or Rut NG-14, is used as the xylanase-producing microorganism to be cultivated.

6. A process according to any one of claims 1 to 5, wherein lactose, cellulose, xylans, wheat bran, glucose syrup and spray-dried corn steep liquor, preferably lactose, cellulose and oat glume xylan, are used as additional carbon sources for the nutrient medium.

7. A process according to any one of claims 1 to 6, wherein ammonium sulphate, ammonia water and de-oiled soya meal are used as the nitrogen sources for the nutrient medium.

8. A process according to any one of claims 1 to 7, wherein the nutrient medium also has a phosphorus source, preferably potassium dihydrogen phosphate, and/or salts, preferably magnesium, iron, manganese and zinc sulphate as well as calcium and colbalt chloride.

9. A process according to any one of claims 1 to 8, wherein the pH value and the temperature for the cultivation lies in the range of about 5.5 to about 6.5 and, respectively, in the range of about 28°C to about 34°C.

10. A process according to any one of claims 1 to 9, wherein de-oiled soya meal or soya meal liquor is used as an additional xylanase inductor and as a nitrogen source.

11. A process according to any one of claims 1 to 10, wherein the fed batch technique is used for carrying out the process.

## Revendications

1. Procédé de préparation d'un complexe enzymatique riche en xylanase par culture d'un microorganisme produisant de la xylanase de l'espèce Trichoderma dans un milieu nutritif, **caractérisé en ce que** l'on utilise en tant qu'inducteur de xylanase et simultanément en tant que source de carbone un résidu de distillerie de grains prétraité, le prétraitement consistant en une séparation des fractions solides du résidu de distillerie de grains, une concentration des constituants non volatils par évaporation d'eau et d'autres substances volatiles, ainsi qu'un autoclavage subséquent du concentré de résidu de distillerie de grains ainsi obtenu.

2. Procédé selon la revendication 1, **caractérisé en ce que** la séparation des fractions solides du résidu de distillerie de grains est réalisée par décantation et séparation au moyen d'un séparateur centrifuge, le volume de la fraction contenant des solides à écarter étant d'environ 30% à environ 50% du volume du résidu de distillerie de grains.

3. Procédé selon la revendication 1 ou 2, **caractérisé en ce que** la concentration des constituants non volatils du résidu de distillerie de grains exempt de solides est réalisée au moyen d'un évaporateur sous vide à circulation, à une température d'ébullition située entre environ 40°C et environ 50°C, et à la pression de vapeur correspondant à la température du résidu de distillerie de grains, et l'on réalise une diminution de volume du résidu de distillerie de grains exempt de solides d'environ 80% à environ 90% après environ une heure.

4. Procédé selon l'une quelconque des revendications 1 à 3, **caractérisé en ce que** l'autoclavage du concentré de résidu de distillerie de grains est entrepris pendant au moins environ 30 minutes à environ 121°C.

5. Procédé selon l'une quelconque des revendications 1 à 4, **caractérisé en ce que** l'on utilise en tant que microorganisme producteur de xylanase un mutant de Trichoderma reesei, dérivé de la souche sauvage QM6a, en particulier QM9123, QM9414, M18.2-y, Rut M-7 ou Rut NG-14.

6. Procédé selon l'une quelconque des revendications 1 à 5, **caractérisé en ce que** l'on utilise comme autre source de carbone pour le milieu nutritif du lactose, de la cellulose, des xylanes, du son de blé, du sirop de glucose et de l'eau de trempe de maïs séchée par pulvérisation, de préférence du lactose, de la cellulose et du xylane de glumes d'avoine.

7. Procédé selon l'une quelconque des revendications 1 à 6, **caractérisé en ce que** l'on utilise comme sources d'azote pour le milieu nutritif du sulfate d'ammonium, de l'eau ammoniacale et de la farine de soja déshuilée.

8. Procédé selon l'une quelconque des revendications 1 à 7, **caractérisé en ce que** le milieu nutritif présente aussi une source de phosphore, de préférence du dihydrogénophosphate de potassium, et/ou des sels, de préférence du sulfate de magnésium, de fer, de manganèse et de zinc ainsi que du chlorure de calcium et de cobalt.

9. Procédé selon l'une quelconque des revendications 1 à 8, **caractérisé en ce que** le pH et la température de la culture sont respectivement de l'ordre d'environ 5,5 à environ 6,5 et de l'ordre d'environ 28°C à environ 34°C.

10. Procédé selon l'une quelconque des revendications 1 à 9, **caractérisé en ce que** l'on utilise comme inducteur additionnel de xylanase et comme source d'azote de la farine de soja déshuilée ou un brassin de farine de soja.

11. Procédé selon l'une quelconque des revendications 1 à 10, **caractérisé en ce que** l'on utilise pour l'exécution du procédé la technique fed-batch.
